# EUROPÄISCHE PATENTANMELDUNG

(11) **EP 0 665 018 A1**
(43) Veröffentlichungstag der Anmeldung: **02.08.1995**
(21) Anmeldenummer: 94119812.9
(22) Anmeldetag: 15.12.1994
(51) Int. Cl.: A61K 35/78

(54) **Flüssige Mixtur zur inneren Anwendung für den vorbeugenden Gesundheitsschutz**

(30) Priorität: 20.01.1994 DE 9402223 U
(71) Anmelder: Latta, Bernd, D-33604 Bielefeld (DE)
(72) Erfinder: Latta, Bernd, D-33604 Bielefeld (DE)
(74) Vertreter: Schirmer, Siegfried, Dipl.-Ing.

(57) **Zusammenfassung**

Es wird eine flüssige Mixtur zur inneren Anwendung für den vorbeugenden Gesundheitsschutz vorgeschlagen, die aus einem Gemisch aus zellularflüssiger Bierhefe und Saft aus Lebensmitteln gebildet ist, wobei das Gemisch mit einer neutralen Flüssigkeit verdünnt ist.

Vorteilhafterweise findet ein Saft aus Gräsern, Kräutern, Früchten, Wurzeln, Gemüsen, Samen und Keimlingen Verwendung. Als neutrale Flüssigkeit kann kohlensäurefreies natürliches Mineralwasser dienen.

## Beschreibung

Die Erfindung betrifft eine flüssige MIxtur zur inneren Anwendung für den vorbeugenden Gesundheitsschutz auf der Basis der Wirkstoffe biologischer Naturprodukte, insbesondere zum vorbeugenden Schutz gegen die negativen Folgen der Einwirkung von Strahlen, wie Röntgenstrahlen und dergleichen, auf den menschlichen Organismus.

Zahlreiche Krankheitsbilder des menschlichen Körpers werden durch Mangelerscheinungen, beispielsweise Vitaminmangel hevorgerufen. Die Prophylaxe von Krankheiten durch Stärkung der körpereigenen Abwehrkräfte durch Zufuhr von in der Natur vorkommenden Wirkstoffen erfreut sich mithin in breiten Bevölkerungsschichten zunehmender Beliebtheit. Es sind daher eine Vielzahl von Produkten bekannt, die auf der krankheitsprophylaktischen Wirkung von Vitaminen oder vitaminhaltigen Naturprodukten beruhen und in der Gesundheitspflege eingesetzt werden.

In der Medizin werden andererseits für zahlreiche Untersuchungsmethoden, aber auch zur Strahlentherapie Strahlen, z. B. Röntgenstrahlen eingesetzt, die neben ihrer hervorragenden Bedeutung für die Behandlung von Krankheiten bei zu hohen Dosen auch eine in hohem Maß gesundheitsbeeinträchtigende Wirkung zeigen. Zu derartig schädigenden Strahlen zählen aber auch die durch den Eingriff des Menschen in seine natürliche Umwelt vermehrt auftretenden Strahlen, beispielsweise die ultravioletten Strahlen des Sonnenlichts. Die biologische Wirkung derartiger Strahlen kann in überhöhter Dosis oder bei Langzeiteinwirkung mit Störungen der Funktion und Struktur des Zellgewebes oder von Organen einhergehen und schließlich den Gesamtorganismus in Mitleidenschaft ziehen. Die bekannten Methoden zum Strahlenschutz durch räumliche Distanzierung oder Abdeckung der gefährdeten Körperteile mit strahlenundurchlässigem Material sind aber oftmals nicht zuverlässig anwendbar.

Der Erfindung liegt daher die Aufgabe zugrunde, eine flüssige Mixtur auf der Basis in biologischen Naturprodukten enthaltener Wirkstoffe anzugeben, die sowohl der natürlichen Gesundheitspflege und allgemein dem vorbeugenden Gesundheitsschutz dient, aber auch bei der Prophylaxe von durch Röntgen- und anderen auf den menschlichen Körper wirkenden Strahlenarten verursachten Schäden Verwendung findet.

Erfindungsgemäß wird die Aufgabe mit einer flüssigen Mixtur zu inneren Anwendung beim Menschen durch ein Gemisch aus zellular flüssiger Bierhefe und Saft aus Lebensmitteln, wobei das Gemisch mit einer neutralen Flüssigkeit verdünnt ist, gelöst. In Ausgestaltung der Erfindung findet ein Saft aus Gräsern, Kräutern, Früchten, Wurzeln, Gemüsen, Samen und Keimlingen Verwendung. Vorteilhafterweise wird ein Anteil von 70 bis 85 % zellular flüssiger Bierhefe, 1,5 bis 3 % Grünsaft von Weizengras, 1,5 bis 3 % Grünsaft von Löwenzahnblättern und 15 bis 22 % Zitronen- oder Limonensaft miteinander vermischt.

Weitere Merkmale der Erfindung sind in den übrigen Unteransprüchen aufgezeigt.

Aufgrund der in den genannten Produkten enthaltenen natürlichen Wirkstoffen, z. B. einem breiten Spektrum von Vitamin B und anderen Vitaminen sowie Spurenmetallen, konnte in der erfindungsgemäß vorgeschlagenen Zusammensetzung eine deutliche Wirkung bei der Gesundheitspflege, bei der allgemeinen Vorbeugung gegen verschiedene Krankheitserscheinungen und insbesondere bei der Prophylaxe von durch schädliche Strahlung oder überhöhte Strahlendosen hervorgerufenen Schädigungen des menschlichen Organismus festgestellt werden.

In einer bevorzugten Ausführungsform der Erfindung beträgt der Anteil der zellularen flüssigen Bierhefe 78 %, der an frischen Grünsaft von Weizengras und frischen Grünsaft von Löwenzahn jeweils 1,5 % und der an Zitronen- oder Limonensaft 19 %, wobei die Mixtur in der trinkfertigen Form etwa im Verhältnis 1 : 0,5 bis 1 : 1 mit einer neutralen Flüssigkeit, vorzugsweise kohlensäurefreiem natürlichen Mineralwasser, verdünnbar ist.

Im folgenden wird eine bevorzugte Ausführungsform der vorliegenden Erfindung näher beschrieben.

Etwa 70 Millilitern zellularer flüssiger Bierhefe werden 2 Milliliter frischer Grünsaft von Weizengras und 2 Milliliter frischer Grünsaft der Blätter von Löwenzahn zugemischt und unter Zugabe der Hälfte des Saftes einen sonnengereiften Lemone gut miteinander verrührt. Diese Mixtur wird mit kohlensäurefreiem Mineralwasser auf 110 bis 150 Milliliter aufgefüllt und steht nun als Getränk zur vorbeugenden Gesundheitspflege zur Verfügung.

## Patentansprüche

1. Flüssige Mixtur zur inneren Anwendung für den vorbeugenden Gesundheitsschutz auf der Basis der Wirkstoffe biologischer Naturprodukte, insbesondere zum vorbeugenden Schutz gegen die negativen Folgen der Einwirkung von Strahlen, wie Röntgenstrahlen und dergleichen, auf den menschlichen Organismus, gekennzeichnet durch ein Gemisch aus zellular flüssiger Bierhefe und Saft aus Lebensmitteln, wobei das Gemisch mit einer neutralen Flüssigkeit verdünnt ist.

2. Flüssige Mixtur nach Anspruch 1, gekennzeichnet durch die Verwendung von Saft aus Gräsern, Kräutern, Früchten, Wurzeln, Gemüsen, Samen und Keimlingen.

3. Flüssige Mixtur nach Anspruch 1, gekennzeichnet durch einen Anteil von 70 bis 85 % zellular flüssiger Bierhefe, 1,5 bis 3 % Grünsaft von Weizengras, 1,5 bis 3 % Grünsaft von Löwenzahnblättern und 15 bis 22 % Zitronen- oder Limonensaft verdünnbar ist.

4. Flüssige Mixtur nach Anspruch 1, gekennzeichnet durch eine anteilige Zusammensetzung aus 78 % zellularer flüssiger Bierhefe, jeweils 1,5 % Grünsaft von Weizengras und Grünsaft von Löwenzahnblättern und 19 % Zitronen- oder Limonensaft, wobei das Gemisch etwa im Verhältnis 1 : 0,5 bis 1 : 1 mit einer neutralen Flüssigkeit verdünnt wird.

5. Flüssige Mixtur nach einem der Ansprüche 1 bis 4, dadurch gekennzeichnet, daß die zur Verdünnung verwendete Flüssigkeit kohlensäurefreies natürliches Mineralwasser ist.
